# EUROPEAN PATENT APPLICATION

(11) **EP 4 821 493 A1**
(43) Date of publication of application: **14.10.2026**
(21) Application number: 24900989.5
(22) Date of filing: 02.12.2024
(51) Int. Cl.: A61K 31/165, A61K 39/395, A61K 45/06, A61P 35/00, C07K 16/28, A61K 39/00

(54) **PHARMACEUTICAL COMPOSITION FOR TREATING CANCER COMPRISING SIMULTANEOUS INHIBITOR OF HISTONE DEACETYLASE 6 AND HISTONE DEACETYLASE 10**

(30) Priority: 04.12.2023 KR 20230173635
(71) Applicant: Samsung Life Public Welfare Foundation, Seoul 04348 (KR)
(72) Inventor: CHO, Yong Beom, Seoul 06351 (KR); YU, Ah Ran, Seoul 06351 (KR)
(74) Representative: Plougmann Vingtoft a/s
(86) International application number: PCT/KR2024/019423
(87) International publication number: WO 2025/121803

(57) **Abstract**

The present invention relates to a pharmaceutical composition for treating cancer, comprising a dual inhibitor of histone deacetylase 6 (HDAC6) and histone deacetylase 10 (HDAC10); an anticancer adjuvant for cancer immunotherapy; a composition for increasing PD-L1 expression in cancer cells; a method for treating cancer using a dual inhibitor of HDAC6 and HDAC10; a method for increasing PD-L1 expression in cancer cells; a use of a dual inhibitor of HDAC6 and HDAC10 for treating cancer; a use thereof as an anticancer adjuvant for cancer immunotherapy; and a use thereof for increasing PD-L1 expression in cancer cells.

## Description

### TECHNICAL FIELD

This patent application claims priority to Korean Patent Application No. 10-2023-0173635, filed with the Korean Intellectual Property Office on December 4, 2023, the disclosure of which is incorporated herein by reference.

The present invention was made with support from the Ministry of Health and Welfare under Project Identification No. 1465039353 and Project No. HR20C0025020023. The research management agency for the project is the Korea Health Industry Development Institute, the research program is "Research-Driven Hospital R&D," the title of the research project is "Development of biomarkers and novel targets for immune cell therapy based on an ultra-precise multi-omics analysis system," the host institution is Samsung Medical Center, and the research period is from January 1, 2023 to December 31, 2023.

In addition, the present invention was made with support from the Ministry of Science and ICT under Project Identification No. 1711188753 and Project No. 2020R1A2C2006408. The research management agency for the project is the National Research Foundation of Korea, the research program is "Basic Research Program for Individuals," the title of the research project is "Development of immune microenvironment modulation technology through macrophage-mediated immune profiling of colorectal cancer patients," the host institution is Samsung Medical Center, and the research period is from March 1, 2023 to February 29, 2024.

The present invention relates to a pharmaceutical composition for treating cancer, comprising a dual inhibitor of histone deacetylase 6 (hereinafter, HDAC6) and histone deacetylase 10 (hereinafter, HDAC10); an anticancer adjuvant for cancer immunotherapy; a composition for increasing PD-L1 expression in cancer cells; a method for treating cancer using a dual inhibitor of HDAC6 and HDAC10; a method for increasing PD-L1 expression in cancer cells; a use of a dual inhibitor of HDAC6 and HDAC10 for treating cancer; a use thereof as an anticancer adjuvant for cancer immunotherapy; and a use thereof for increasing PD-L1 expression in cancer cells.

### BACKGROUND

Histone deacetylases (hereinafter, HDACs) are enzymes that regulate the balance between acetylation and deacetylation of histone and non-histone proteins by promoting hydrolysis of ε-amide bonds of lysine residues, and play an important role in gene expression and differentiation, and in maintaining cellular homeostasis.

Histone deacetylases may be recruited to gene promoters by corepressors or multi-protein transcriptional complexes, where they regulate transcription through chromatin modification without directly binding to DNA.

Such HDACs are involved in regulating numerous cellular processes. Histone acetyltransferases (HATs) and HDACs are known to affect transcriptional activity by acetylating or deacetylating lysine residues at the N-termini of histone proteins, and to regulate post-translational acetylation of at least 50 non-histone proteins, such as α-tubulin, Hsp90, p53, c-Myc, NF-κB, and E2P.

There are 18 encoded human HDACs, which are classified into class I (HDAC 1, 2, 3, and 8), class II (HDAC 4, 5, 6, 7, 9, and 10), class III (SIRT 1-7), and class IV (HDAC11).

Overexpression of HDACs in various cancer cells causes suppression of important growth-inhibitory genes, thereby having a mechanism that promotes cancer cell proliferation. Accordingly, HDACs are important drug targets for the development of anticancer agents, and inhibitors thereof are being actively developed.

HDAC inhibitors are generally classified into four types according to their chemical structures, such as hydroxamic acids, benzamides, cyclic peptides, and short-chain fatty acids. To date, the U.S. FDA has approved four HDAC inhibitors, such as SAHA (vorinostat), FK-228 (romidepsin), PXD101 (belinostat), and LBH589 (panobinostat), as anticancer agents, and the Chinese Food and Drug Administration has approved HBI-8000 (chidamide) as a therapeutic agent for T-cell lymphoma.

In addition, about 20 promising HDAC inhibitors are in clinical or preclinical stages for various cancers; however, most HDAC inhibitors have been found to cause many side effects, including fatigue, nausea, vomiting, and cardiotoxicity.

Furthermore, although many studies have been developing new drugs that may be effective for immunotherapy of colorectal cancer (CRC), unfortunately, in the case of CRC, most patients are of the microsatellite stable (MSS) type, and there is a problem in that they do not respond to immunotherapy because their tumor microenvironment (TME) differs from that of the microsatellite instable (MSI) type.

### SUMMARY

Accordingly, the present inventors investigated whether specific HDAC isoforms can regulate immune-related genes in the tumor microenvironment of colorectal cancer, and whether selective HDAC inhibitors are effective for treating colorectal cancer.

As a result, the present inventors confirmed that HDAC6 and HDAC10 are important epigenetic regulators of PD-L1 in colorectal cancer, and confirmed that treatment with a dual inhibitor of HDAC6 and HDAC10 upregulates PD-L1 expression in colorectal cancer cell lines, thereby confirming that the dual inhibitor is effective for immunotherapy in colorectal cancer patients.

Accordingly, an object of the present invention is to provide a pharmaceutical composition for treating cancer, comprising a dual inhibitor of Histone Deacetylase 6 (hereinafter, HDAC6) and Histone Deacetylase 10 (hereinafter, HDAC10).

Another object of the present invention is to provide an anticancer adjuvant for cancer immunotherapy, comprising a dual inhibitor of HDAC6 and HDAC10.

Still another object of the present invention is to provide a composition for increasing PD-L1 expression in cancer cells, comprising a dual inhibitor of HDAC6 and HDAC10.

Still another object of the present invention relates to a method for treating cancer using a dual inhibitor of HDAC6 and HDAC10.

Still another object of the present invention is to provide a method for increasing PD-L1 expression in cancer cells using a dual inhibitor of HDAC6 and HDAC10.

Still another object of the present invention relates to a use of a dual inhibitor of HDAC6 and HDAC10 for treating cancer.

Still another object of the present invention relates to a use of a dual inhibitor of HDAC6 and HDAC10 as an anticancer adjuvant for cancer immunotherapy.

Still another object of the present invention relates to a use of a dual inhibitor of HDAC6 and HDAC10 for increasing PD-L1 expression in cancer cells.

### BRIEF DESCRIPTION OF DRAWINGS

FIG. 1 shows results of measuring the IC₅₀ value of Bufexamac, a dual inhibitor of HDAC6 and HDAC10, according to an embodiment of the present invention.
FIG. 2 shows results of measuring amounts of H3K9ac, H3, and β-actin after treatment with Bufexamac, a dual inhibitor of HDAC6 and HDAC10, according to an embodiment of the present invention, at different concentrations for 48 hours.
FIGS. 3a to 3b show results of measuring morphological changes in M2 macrophages and changes in mRNA expression of CD206, an M2 marker, after treatment with Bufexamac, a dual inhibitor of HDAC6 and HDAC10, according to an embodiment of the present invention, at different concentrations for 48 hours.
FIGS. 4a to 4c show results of measuring tumor volume and weight, and mouse body weight, when Bufexamac, a dual inhibitor of HDAC6 and HDAC10, and an anti-PD-L1 antibody were each administered alone or in combination in an animal experiment according to an embodiment of the present invention.
FIG. 5a shows results of measuring changes in CD3+ T cells, CD4+ T cells, CD8+ T cells, and regulatory T cells in lymph node cells when Bufexamac, a dual inhibitor of HDAC6 and HDAC10, and an anti-PD-L1 antibody were each administered alone or in combination according to an embodiment of the present invention.
FIG. 5b shows results of measuring changes in monocytic myeloid-derived suppressor cells (M-MDSCs), polymorphonuclear leukocyte myeloid-derived suppressor cells (PMN-MDSCs), macrophages, M1 macrophages, and M2 macrophages in bone marrow cells when Bufexamac, a dual inhibitor of HDAC6 and HDAC10, and an anti-PD-L1 antibody were each administered alone or in combination according to an embodiment of the present invention.
FIG. 6 shows results confirming an increase in mRNA expression of PD-L1 by Bufexamac, a dual inhibitor of HDAC6 and HDAC10, in the human MSS CRC cell line SW620 according to an embodiment of the present invention.
FIG. 7 shows results confirming an increase in mRNA expression of PD-L1 by Bufexamac, a dual inhibitor of HDAC6 and HDAC10, in the mouse MSS CRC cell line CT26 according to an embodiment of the present invention.

### DETAILED DESCRIPTION

The present invention relates to a pharmaceutical composition for treating cancer, comprising a dual inhibitor of histone deacetylase 6 (Histone deacetylase 6; hereinafter, HDAC6) and histone deacetylase 10 (Histone deacetylase 10; hereinafter, HDAC10); an anticancer adjuvant for cancer immunotherapy; a composition for increasing PD-L1 expression in cancer cells; a method for treating cancer using a dual inhibitor of HDAC6 and HDAC10; a method for increasing PD-L1 expression in cancer cells; a use of a dual inhibitor of HDAC6 and HDAC10 for treating cancer; a use thereof as an anticancer adjuvant for cancer immunotherapy; and a use thereof for increasing PD-L1 expression in cancer cells.

Hereinafter, the present invention will be described in more detail.

One aspect of the present invention relates to a pharmaceutical composition for treating cancer, comprising a dual inhibitor of histone deacetylase 6 (Histone deacetylase 6, HDAC6) and histone deacetylase 10 (Histone deacetylase 10, HDAC10).

In the present invention, the dual inhibitor of HDAC6 and HDAC10 may be Bufexamac, but is not limited thereto.

In the present invention, the pharmaceutical composition for treating cancer may further comprise an anti-PD-L1 antibody.

As used herein, the term "PD-L1" refers to programmed death-ligand 1, which is also referred to as CD274 or B7-H1. PD-L1 is present on the surface of cells and functions to prevent T cells from attacking, and some tumor cells have a large amount of PD-L1, thereby allowing the tumor cells to evade the immune system, which is the body's natural defense system.

As used herein, the term "anti-PD-L1 antibody" means an antibody capable of specifically binding to PD-L1.

In the present invention, the anti-PD-L1 antibody may be at least one selected from the group consisting of durvalumab, atezolizumab, avelumab, or any fragment, derivative, conjugate, variant, radioisotope-labeled complex, or biosimilar thereof, but is not limited thereto.

In the present invention, the cancer may be at least one selected from the group consisting of colorectal cancer, pancreatic cancer, gastric cancer, and lung cancer.

In the present invention, the pharmaceutical composition for treating cancer may be one in which a target cell line is an MSS cell line, and may be, for example, SW620, SW480, LS513, HT29, LS1034, or CT26.

In the present invention, the pharmaceutical composition for treating cancer may further comprise one or more additives selected from the group consisting of suitable carriers, excipients, disintegrants, sweeteners, coating agents, swelling agents, lubricants, glidants, flavoring agents, antioxidants, buffers, bacteriostatic agents, diluents, dispersants, surfactants, binders, and lubricants, which are commonly used in the preparation of pharmaceutical compositions.

In the present invention, the pharmaceutical composition may further comprise a pharmaceutically acceptable carrier.

As used herein, the term "pharmaceutically acceptable" means one that is commonly used in the pharmaceutical field, does not stimulate an organism upon administration, and does not impair the biological activity and properties of the administered compound.

In the present invention, any carrier commonly used in the art may be used as the carrier. Non-limiting examples of the carrier may include saline, sterile water, Ringer's solution, buffered saline, albumin injection solution, lactose, dextrose, sucrose, sorbitol, mannitol, xylitol, erythritol, maltitol, maltodextrin, glycerol, ethanol, or a combination thereof.

In the present invention, the pharmaceutical composition for treating cancer may be administered to a subject in a conventional manner through an intravenous, intraarterial, intraperitoneal, intramuscular, intrasternal, transdermal, intranasal, inhalation, topical, rectal, oral, intraocular, or intradermal route.

In the present invention, the dosage of the pharmaceutical composition for treating cancer may vary depending on the condition and body weight of the subject, the type and severity of the disease, the drug form, and the administration route and period, and may be appropriately selected by a person skilled in the art. For example, the daily dose may be 0.01 to 200 mg/kg, 0.1 to 200 mg/kg, 0.1 to 100 mg/kg, or 0.1 to 50 mg/kg. The administration may be performed once a day or in divided doses several times a day, and the scope of the present invention is not limited thereby.

In the present invention, the subject may be a mammal, for example, a human or a mouse, but is not limited thereto.

Another aspect of the present invention relates to an anticancer adjuvant for cancer immunotherapy, comprising a dual inhibitor of HDAC6 and HDAC10.

In the present invention, the dual inhibitor of HDAC6 and HDAC10 may be Bufexamac, but is not limited thereto.

In the present invention, the anticancer adjuvant for cancer immunotherapy may further comprise an anti-PD-L1 antibody.

In the present invention, the cancer may be at least one selected from the group consisting of colorectal cancer, pancreatic cancer, gastric cancer, and lung cancer.

In the present invention, the anticancer adjuvant for cancer immunotherapy may be one in which a target cell line is an MSS cell line, and may be, for example, SW620, SW480, LS513, HT29, LS1034, or CT26.

In the present invention, the anticancer adjuvant for cancer immunotherapy may further comprise one or more additives selected from the group consisting of suitable carriers, excipients, disintegrants, sweeteners, coating agents, swelling agents, lubricants, glidants, flavoring agents, antioxidants, buffers, bacteriostatic agents, diluents, dispersants, surfactants, binders, and lubricants, which are commonly used in the preparation of pharmaceutical compositions.

In the present invention, the anticancer adjuvant for immunotherapy may be administered to a subject in a conventional manner through an intravenous, intraarterial, intraperitoneal, intramuscular, intrasternal, transdermal, intranasal, inhalation, topical, rectal, oral, intraocular, or intradermal route.

In the present invention, the dosage of the anticancer adjuvant for immunotherapy may vary depending on the condition and body weight of the subject, the type and severity of the disease, the drug form, and the administration route and period, and may be appropriately selected by a person skilled in the art. For example, the daily dose may be 0.01 to 200 mg/kg, 0.1 to 200 mg/kg, 0.1 to 100 mg/kg, or 0.1 to 50 mg/kg. The administration may be performed once a day or in divided doses several times a day, and the scope of the present invention is not limited thereby.

In the present invention, the subject may be a mammal, for example, a human or a mouse, but is not limited thereto.

Still another aspect of the present invention relates to a composition for increasing PD-L1 expression in cancer cells, comprising a dual inhibitor of HDAC6 and HDAC10.

In the present invention, the dual inhibitor of HDAC6 and HDAC10 may be Bufexamac, but is not limited thereto.

In the present invention, the cancer may be at least one selected from the group consisting of colorectal cancer, pancreatic cancer, gastric cancer, and lung cancer.

In the present invention, the composition for increasing PD-L1 expression in cancer cells may be one in which a target cell line is an MSS cell line, and may be, for example, SW620, SW480, LS513, HT29, LS1034, or CT26.

Still another aspect of the present invention relates to a method for treating cancer, comprising administering a dual inhibitor of HDAC6 and HDAC10 to a subject.

In the present invention, the dual inhibitor of HDAC6 and HDAC10 may be Bufexamac, but is not limited thereto.

In the present invention, the method for treating cancer may comprise administering an anti-PD-L1 antibody to the subject simultaneously or sequentially with the dual inhibitor of HDAC6 and HDAC10.

In the present invention, the cancer may be at least one selected from the group consisting of colorectal cancer, pancreatic cancer, gastric cancer, and lung cancer.

In the present invention, the method for treating cancer may be one in which a target cell line is an MSS cell line, and may be, for example, SW620, SW480, LS513, HT29, LS1034, or CT26.

In the present invention, the subject may be a mammal, for example, a human or a mouse, but is not limited thereto.

In the present invention, the administration may be performed through an intravenous, intraarterial, intraperitoneal, intramuscular, intrasternal, transdermal, intranasal, inhalation, topical, rectal, oral, intraocular, or intradermal route.

In the present invention, the dosage may vary depending on the condition and body weight of the subject, the type and severity of the disease, the drug form, and the administration route and period, and may be appropriately selected by a person skilled in the art. For example, the daily dose may be 0.01 to 200 mg/kg, 0.1 to 200 mg/kg, 0.1 to 100 mg/kg, or 0.1 to 50 mg/kg. The administration may be performed once a day or in divided doses several times a day, and the scope of the present invention is not limited thereby.

In the present invention, the method for treating cancer may comprise administering the dual inhibitor of HDAC6 and HDAC10 to the subject at intervals of 1 to 3 days, or 1 to 2 days, for example, at intervals of 1 day, and simultaneously administering the anti-PD-L1 antibody at intervals of 2 to 4 days, or 2 to 3 days, for example, at intervals of 3 days, but is not limited thereto.

In the present invention, the method for treating cancer may comprise administering the dual inhibitor of HDAC6 and HDAC10 to the subject at a dose per administration of 0.01 to 200 mg/kg, 0.1 to 200 mg/kg, 1 to 200 mg/kg, 10 to 200 mg/kg, 50 to 200 mg/kg, 75 to 200 mg/kg, 100 to 200 mg/kg, 125 to 200 mg/kg, or 150 to 200 mg/kg, for example, 150 mg/kg, but is not limited thereto.

In the present invention, the method for treating cancer may comprise administering the dual inhibitor of HDAC6 and HDAC10 to the subject at a dose per administration of 0.01 to 200 mg/kg, 0.1 to 200 mg/kg, 1 to 150 mg/kg, 1 to 100 mg/kg, 1 to 75 mg/kg, 1 to 50 mg/kg, 10 to 150 mg/kg, 10 to 100 mg/kg, 10 to 75 mg/kg, 10 to 50 mg/kg, 25 to 150 mg/kg, 25 to 100 mg/kg, 25 to 75 mg/kg, or 25 to 50 mg/kg, for example, 50 mg/kg, and administering the anti-PD-L1 antibody at a dose per administration of 0.01 to 200 mg/kg, 0.1 to 200 mg/kg, 0.1 to 100 mg/kg, 0.1 to 50 mg/kg, or 0.1 to 20 mg/kg, for example, 10 mg/kg, but is not limited thereto.

Still another aspect of the present invention relates to a method for increasing PD-L1 expression in cancer cells using a dual inhibitor of HDAC6 and HDAC10.

In the present invention, the dual inhibitor of HDAC6 and HDAC10 may be Bufexamac, but is not limited thereto.

In the present invention, the cancer may be at least one selected from the group consisting of colorectal cancer, pancreatic cancer, gastric cancer, and lung cancer.

In the present invention, the method for increasing PD-L1 expression in cancer cells may be one in which a target cell line is an MSS cell line, and may be, for example, SW620, SW480, LS513, HT29, LS1034, or CT26.

Still another aspect of the present invention relates to a use of a dual inhibitor of HDAC6 and HDAC10 for treating cancer.

Still another aspect of the present invention relates to a use of a dual inhibitor of HDAC6 and HDAC10 as an anticancer adjuvant for cancer immunotherapy.

Still another aspect of the present invention relates to a use of a dual inhibitor of HDAC6 and HDAC10 for increasing PD-L1 expression in cancer cells.

Still another aspect of the present invention relates to a combination kit for preventing or treating cancer, comprising (i) a dual inhibitor of HDAC6 and HDAC10 and (ii) an anti-PD-L1 antibody.

In one embodiment of the present invention, (i) the dual inhibitor of HDAC6 and HDAC10 and (ii) the anti-PD-L1 antibody may be contained in the same container or in different containers.

The kit may further comprise, in addition to the active ingredients, i.e., (i) the dual inhibitor of HDAC6 and HDAC10 and (ii) the anti-PD-L1 antibody, containers, materials, package inserts, and the like generally used in the art and suitable for use as a combination kit for preventing or treating cancer.

The containers may include vials, syringes, bottles, and the like, but are not necessarily limited thereto.

The materials may include diluents such as physiological saline, filters, IV bags and lines, needles, syringes, and the like, but are not necessarily limited thereto.

The package insert may be instructions for using (i) the dual inhibitor of HDAC6 and HDAC10 and (ii) the anti-PD-L1 antibody in combination to treat or delay the progression of cancer, or instructions for treating a subject for cancer using (i) the dual inhibitor of HDAC6 and HDAC10 and (ii) the anti-PD-L1 antibody.

In the present invention, the dual inhibitor of HDAC6 and HDAC10 may be Bufexamac, but is not necessarily limited thereto.

In the present invention, the anti-PD-L1 antibody may be at least one selected from the group consisting of durvalumab, atezolizumab, avelumab, or any fragment, derivative, conjugate, variant, radioisotope-labeled complex, or biosimilar thereof, but is not limited thereto.

In the present invention, (i) the dual inhibitor of HDAC6 and HDAC10 and (ii) the anti-PD-L1 antibody may be administered simultaneously or sequentially.

As used herein, the term "simultaneously" means that two agents or pharmaceutical compositions are administered at the same time point.

As used herein, the term "sequentially" means that active ingredients or pharmaceutical compositions are administered in sequence without being administered simultaneously. For example, "sequentially" administered may mean that, when the circulating half-life of the first-administered agent is sufficiently long such that the two agents can be present simultaneously in therapeutically effective amounts, one agent/pharmaceutical composition is administered within 1 minute, 5 minutes, 10 minutes, 30 minutes, or several hours after administration of the other agent/pharmaceutical composition, for example, within 1 hour, 2 hours, 3 hours, 4 hours, 6 hours, 8 hours, 12 hours, 24 hours, 48 hours, or 72 hours, but is not necessarily limited thereto. The delay in administration time between the components may vary depending on the exact properties, interactions, and respective half-lives of the components.

In one embodiment of the present invention, (i) the dual inhibitor of HDAC6 and HDAC10 and (ii) the anti-PD-L1 antibody may be administered at the same time point or at different time intervals.

In one embodiment of the present invention, (i) the dual inhibitor of HDAC6 and HDAC10 and (ii) the anti-PD-L1 antibody may be administered by the same route of administration or by different routes of administration.

In the present invention, the cancer may be at least one selected from the group consisting of colorectal cancer, pancreatic cancer, gastric cancer, and lung cancer.

Hereinafter, the present invention will be described in more detail with reference to the following Examples. However, these Examples are provided only for illustrating the present invention, and the scope of the present invention is not limited by these Examples.

### [EXPERIMENTAL EXAMPLE 1] Measurement of IC50 Concentration

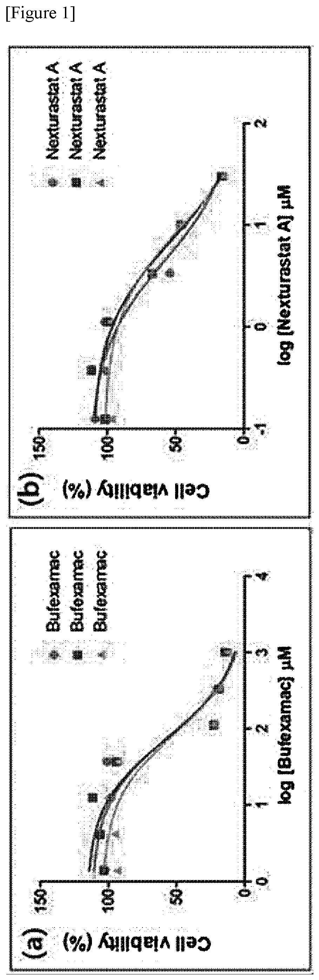

The IC₅₀ concentration is a concentration at which half of the cells die, and the IC₅₀ value was measured by performing a WST assay under the conditions shown in Table 1 below. Specifically, after treating the CT26 colorectal cancer cell line with an HDAC6 inhibitor (Nexturastat A) and a dual inhibitor of HDAC6 and HDAC10 (Bufexamac) at different concentrations for 72 hours, the concentration at which half of the cells died was determined, and the results are shown in FIG. 1.

**TABLE 1**

| 1. IC50 (WST assay) | |
|---|---|
| Cell line | CT26 |
| Cell number | 1×10⁴ |
| HDAC6 inhibitor (Nexturastat A) | 30 µM ~ 3-fold dilution |
| HDAC class IIb inhibitor (Bufexamac) | 1 mM ~ 3-fold dilution |
| Incubation time | 72 hrs |

As shown in FIG. 1, the IC₅₀ of Bufexamac was determined to be 107.4 µM, and the IC₅₀ of Nexturastat A was determined to be 12.8 µM. Thus, the IC₅₀ value of Bufexamac was confirmed to be about 7 times higher than that of Nexturastat A. This indicates that Bufexamac has lower drug properties than Nexturastat A, which has excellent selectivity, and also indicates that animal experiments should be conducted using a higher concentration of Bufexamac than Nexturastat A.

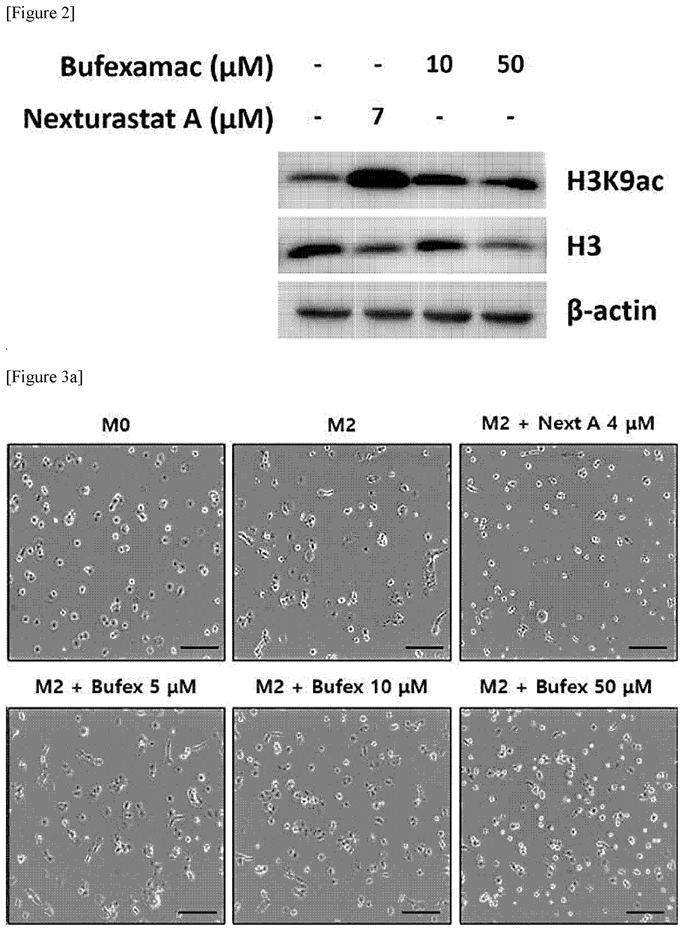

[EXPERIMENTAL EXAMPLE 2] Confirmation of HDAC Inhibitory Effect The HDAC inhibitory effect was confirmed by Western blot under the conditions shown in Table 2 below. Specifically, after treatment with an HDAC6 inhibitor (Nexturastat A) and a dual inhibitor of HDAC6 and HDAC10 (Bufexamac) at different concentrations for 48 hours, the amounts of H3K9ac, H3, and β-actin were measured, and the results are shown in FIG. 2.

**TABLE 2**

| 2. HDAC inhibitory effect (Histone acetylation; Western blot) | |
|---|---|
| Cell line | CT26 |
| Cell number | 2×10⁵ |
| HDAC6 inhibitor (Nexturastat A) | 7 µM |
| HDAC class IIb inhibitor (Bufexamac) | 10 µM, 50 µM |
| Incubation time | 48 hrs |

As shown in FIG. 2, it was confirmed that when the function of HDAC was lost due to the HDAC inhibitor, acetylation of H3K9 was increased. As confirmed in FIG. 1, in the case of Bufexamac, which has lower drug properties than Nexturastat A, the degree of H3K9 acetylation was weak, but was confirmed to be increased compared with the control group. Thus, it was confirmed that Bufexamac also inhibits intracellular HDAC.

### [EXPERIMENTAL EXAMPLE 3] Confirmation of M2 Macrophage Modulation

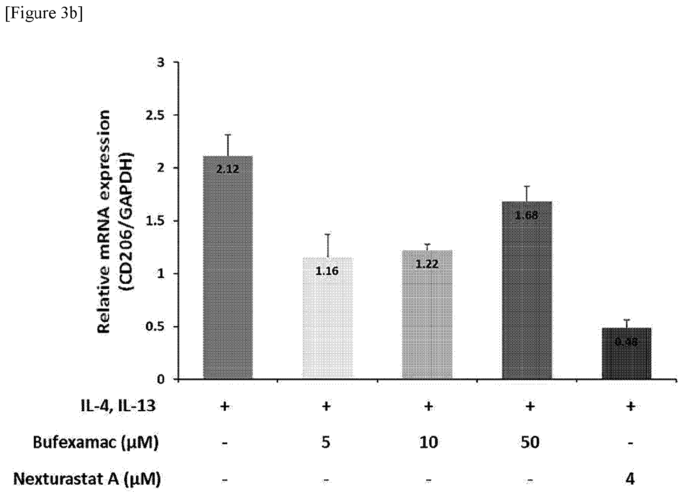

The M2 macrophage-modulating effect was confirmed by RT-PCR under the conditions shown in Table 3 below. Specifically, THP-1, a human monocytic cell line, was stimulated with PMA to differentiate into macrophages, and then treated with IL-4 and IL-13 to differentiate into M2 macrophages. After the differentiated M2 macrophages were treated with an HDAC6 inhibitor (Nexturastat A) and a dual inhibitor of HDAC6 and HDAC10 (Bufexamac) at different concentrations for 48 hours, changes in the M2 macrophages were measured, and the results are shown in FIGS. 3a and 3b.

**TABLE 3**

| 3. M2 macrophage modulation (RT-PCR; CD206) | |
|---|---|
| Cell line | THP-1 (w/PMA) |
| Cell number | 3×10⁵ |
| M2 differentiation | using IL-4, IL-13 |
| HDAC6 inhibitor (Nexturastat A) | 4 µM |
| HDAC class IIb inhibitor (Bufexamac) | 5, 10, 50 µM |
| Incubation time | 48 hrs |

As shown in FIG. 3a, M2 macrophages exhibited an elongated shape compared with M0 macrophages, whereas cells having a round shape similar to M0 macrophages were observed under the conditions treated with 4 µM Nexturastat A and 50 µM Bufexamac. This indicates that the M2 macrophages lost their M2 macrophage phenotype due to the HDAC inhibitors, Nexturastat A and Bufexamac.

In addition, as shown in FIG. 3b, RNA was isolated under the same conditions, and changes in RNA expression of CD206, an M2 macrophage marker, were examined. As a result, it was confirmed that expression of CD206 was decreased by the HDAC inhibitors, Nexturastat A and Bufexamac. This indicates that the HDAC inhibitors, Nexturastat A and Bufexamac, reduce M2 macrophages.

### [EXPERIMENTAL EXAMPLE 4] Animal Experiment Using Dual Inhibitor of HDAC6 and HDAC10

Tumors were formed by subcutaneously injecting 1×10⁶ mouse MSS colorectal cancer cell line CT26 cells into BALB/c mice.

The animal experiment using the dual inhibitor of HDAC6 and HDAC10 was conducted with a total of four groups: a control group, an anti-PD-L1 10 mg/kg group, a Bufexamac 50 mg/kg group, and a Bufexamac 50 mg/kg + anti-PD-L1 group.

Bufexamac (Selleckchem) was used as the dual inhibitor of HDAC6 and HDAC10, and #BE0101 (Bio X Cell) was used as anti-PD-L1 (anti-PD-L1 antibody). CT26 cells (1×10⁶) were used as the cell line, and BALB/c mice were used as the mice. Five mice were assigned to each of the control group, the anti-PD-L1 10 mg/kg group, the Bufexamac 50 mg/kg group, and the Bufexamac 50 mg/kg + anti-PD-L1 group. The treatment conditions were as follows.

### (Treatment Conditions)

- Anti-PD-L1 10 mg/kg group: intraperitoneal administration once every 3 days
- Bufexamac 50 mg/kg group: daily intraperitoneal administration
- Bufexamac 50 mg/kg + anti-PD-L1 10 mg/kg group: intraperitoneal administration of anti-PD-L1 once every 3 days and daily intraperitoneal administration of Bufexamac

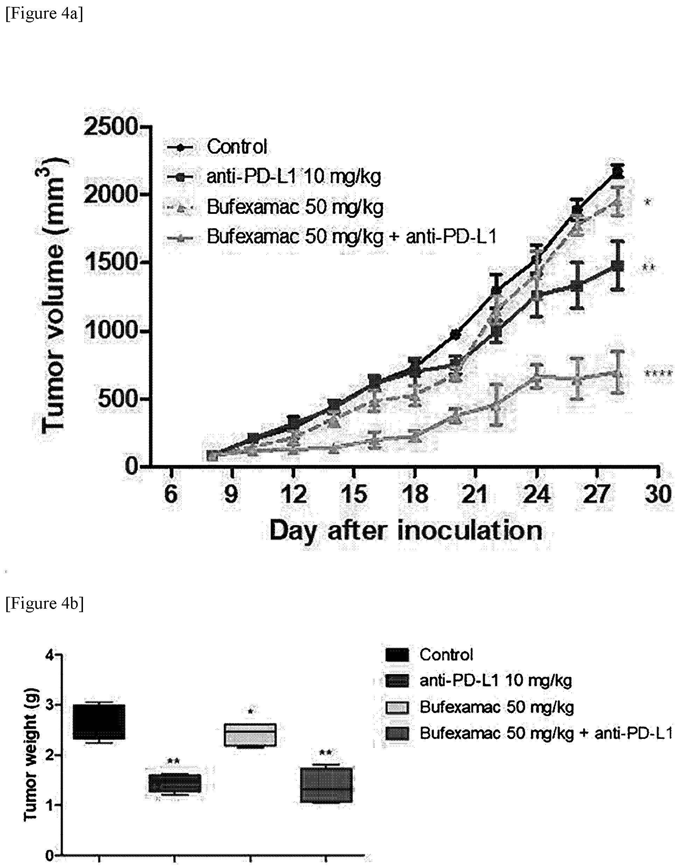

Tumor volume and weight were measured in each group, and the results are shown in FIGS. 4a to 4b. As can be seen from FIGS. 4a to 4b, when the dual inhibitor of HDAC6 and HDAC10 (Bufexamac) was administered alone at 50 mg/kg, there was no significant change in tumor volume and weight compared with the control group. However, when the dual inhibitor of HDAC6 and HDAC10 was administered in combination with the anti-PD-L1 antibody, a markedly superior tumor volume- and weight-reducing effect was observed.

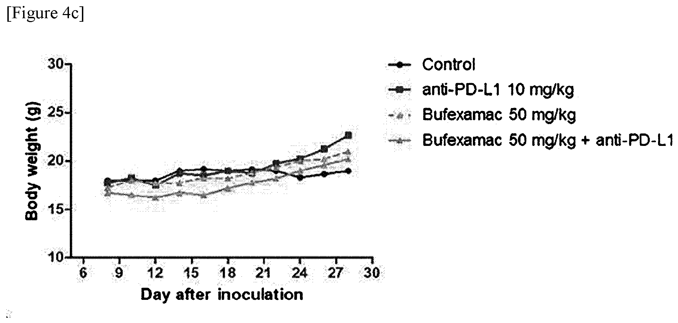

In addition, in the animal experiment, the body weight of the mice was measured when the dual inhibitor of HDAC6 and HDAC10 was administered, and the results are shown in FIG. 4c. From this, it was confirmed that the body weight of the animals was not reduced by the drug.

### [EXPERIMENTAL EXAMPLE 5] Analysis of Lymph Node Cells

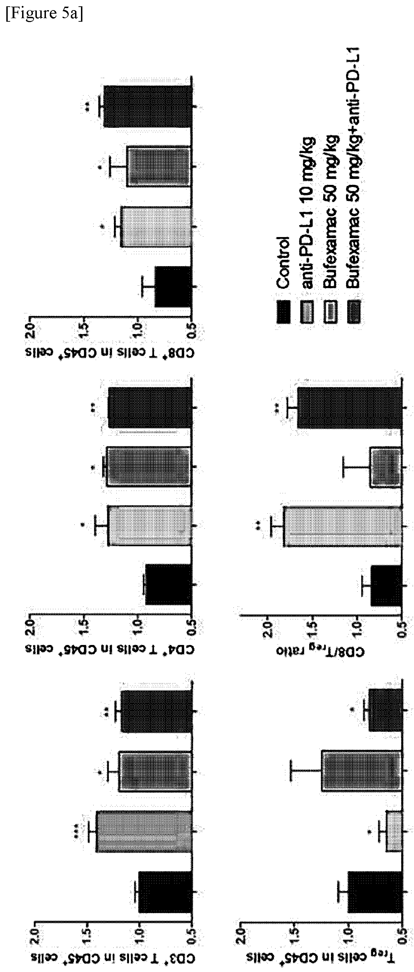

In addition, in the animal experiment, when the dual inhibitor of HDAC6 and HDAC10, the anti-PD-L1 antibody, and the dual inhibitor of HDAC6 and HDAC10 together with the anti-PD-L1 antibody were administered, changes in CD3+ T cells, CD4+ T cells, CD8+ T cells, and regulatory T cells in lymph node cells were measured by flow cytometry (FACS), and the results are shown in FIG. 5a.

As can be seen from FIG. 5a, CD8+ T cells were confirmed to increase particularly more in the group treated with the anti-PD-L1 antibody together with the dual inhibitor of HDAC6 and HDAC10 than in the groups treated with either the anti-PD-L1 antibody or the dual inhibitor of HDAC6 and HDAC10 alone. This indicates that the dual inhibitor of HDAC6 and HDAC10 may induce infiltration of CD8+ T cells into tumors when used in combination with an immune checkpoint inhibitor.

In addition, when the dual inhibitor of HDAC6 and HDAC10 was administered together with the anti-PD-L1 antibody, regulatory T cells (Tregs) were confirmed to be decreased.

Furthermore, the CD8/Treg ratio was confirmed to increase when the dual inhibitor of HDAC6 and HDAC10 was administered together with the anti-PD-L1 antibody. This indicates that, due to the combined administration of the dual inhibitor of HDAC6 and HDAC10 and the anti-PD-L1 antibody, regulatory T cells, which function to suppress T cells, are decreased, while CD8+ T cells, which function to attack tumors, are increased, thereby inducing a change in the intratumoral immune environment.

### [EXPERIMENTAL EXAMPLE 6] Analysis of Bone Marrow Cells

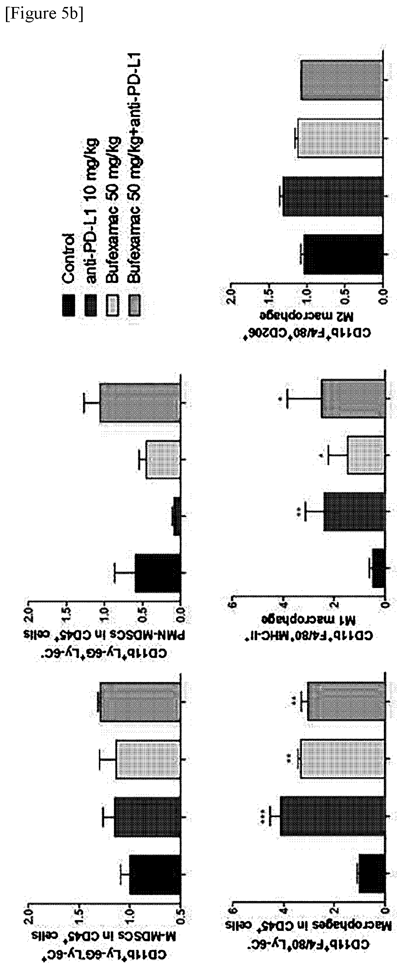

In addition, in the animal experiment, when the dual inhibitor of HDAC6 and HDAC10, the anti-PD-L1 antibody, and the dual inhibitor of HDAC6 and HDAC10 together with the anti-PD-L1 antibody were administered, changes in monocytic myeloid-derived suppressor cells (M-MDSCs), polymorphonuclear leukocyte myeloid-derived suppressor cells (PMN-MDSCs), macrophages, M1 macrophages, and M2 macrophages in bone marrow cells were measured by flow cytometry (FACS), and the results are shown in FIG. 5b.

As can be seen from FIG. 5b, in the case of myeloid-derived suppressor cells (MDSCs) and M2 macrophages, there was no change caused by the dual inhibitor of HDAC6 and HDAC10, and the distribution of macrophages was increased in all groups. M1 macrophages were confirmed to increase compared with other groups when the dual inhibitor of HDAC6 and HDAC10 was administered alone or together with the anti-PD-L1 antibody. This indicates that M1 macrophages, which play a pro-inflammatory role, are increased, thereby likewise inducing a change in the intratumoral immune environment.

### [EXPERIMENTAL EXAMPLE 7] Confirmation of Increase in PD-L1 Expression by Dual Inhibitor of HDAC6 and HDAC10 in MSS Colorectal Cancer

A dual inhibitor of HDAC6 and HDAC10 (Bufexamac) was treated to human and mouse MSS CRC cell lines, RNA was then isolated, and an increase in PD-L1 expression was confirmed by qRT-PCR. GAPDH was used as a control gene.

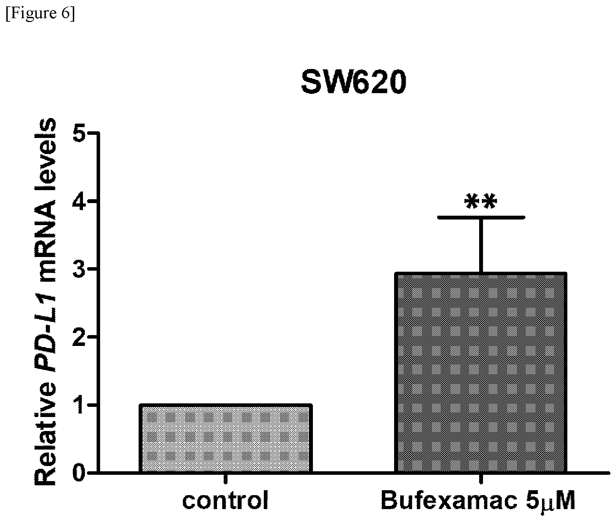

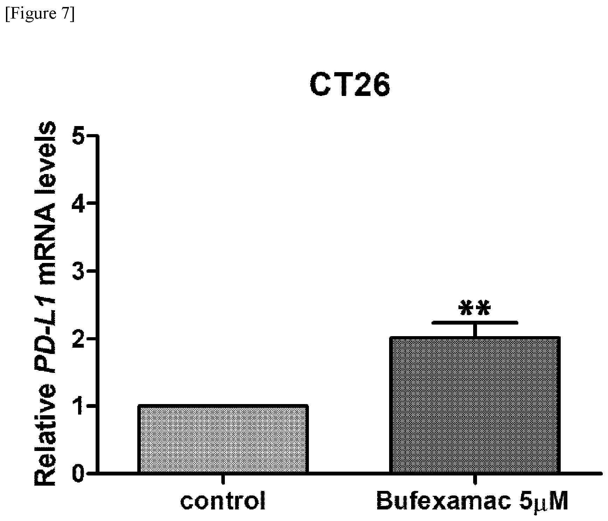

As can be seen from FIGS. 6 and 7, in the human MSS CRC cell line SW620 and the mouse MSS CRC cell line CT26, treatment with 5 µM Bufexamac, a dual inhibitor of HDAC6 and HDAC10, statistically significantly increased mRNA expression of PD-L1 compared with the control group.

From the above results, the present inventors expect that the synergistic effect on anticancer activity against MSS colorectal cancer upon combined treatment with the dual inhibitor of HDAC6 and HDAC10 and the anti-PD-L1 antibody, as compared with single treatment, is achieved by the dual inhibitor of HDAC6 and HDAC10 inducing an increase in PD-L1 expression in MSS colorectal cancer, and by the anti-PD-L1 antibody responding to the increased expression of PD-L1.

### [CONCLUSION]

It was confirmed that the tumor growth inhibitory effect was superior when the dual inhibitor of HDAC6 and HDAC10 was administered together with an anti-PD-L1 antibody, compared with when the dual inhibitor of HDAC6 and HDAC10 was administered alone. Accordingly, the pharmaceutical composition comprising the dual inhibitor of HDAC6 and HDAC10 according to the present invention is expected to be usable as an effective composition for treating cancer.

## Claims

1. A pharmaceutical composition for treating cancer, comprising a dual inhibitor of histone deacetylase 6 (HDAC6) and histone deacetylase 10 (HDAC10).

2. The pharmaceutical composition for treating cancer according to claim 1, wherein the pharmaceutical composition for treating cancer further comprises an anti-PD-L1 antibody.

3. The pharmaceutical composition for treating cancer according to claim 1, wherein the cancer is at least one selected from the group consisting of colorectal cancer, pancreatic cancer, gastric cancer, and lung cancer.

4. An anticancer adjuvant for cancer immunotherapy, comprising a dual inhibitor of histone deacetylase 6 (HDAC6) and histone deacetylase 10 (HDAC10).

5. The anticancer adjuvant for cancer immunotherapy according to claim 4, wherein the anticancer adjuvant for cancer immunotherapy further comprises an anti-PD-L1 antibody.

6. The anticancer adjuvant for cancer immunotherapy according to claim 4, wherein the cancer is at least one selected from the group consisting of colorectal cancer, pancreatic cancer, gastric cancer, and lung cancer.

7. A composition for increasing PD-L1 expression in cancer cells, comprising a dual inhibitor of histone deacetylase 6 (HDAC6) and histone deacetylase 10 (HDAC10).

8. The composition for increasing PD-L1 expression in cancer cells according to claim 7, wherein the cancer is at least one selected from the group consisting of colorectal cancer, pancreatic cancer, gastric cancer, and lung cancer.

9. A method for increasing PD-L1 expression in cancer cells, comprising contacting to a subject with a dual inhibitor of histone deacetylase 6 (HDAC6) and histone deacetylase 10 (HDAC10).

10. The method for increasing PD-L1 expression in cancer cells according to claim 9, wherein the cancer is at least one selected from the group consisting of colorectal cancer, pancreatic cancer, gastric cancer, and lung cancer.
